(19) Europäisches Patentamt
European Patent Office
Office européen des brevets

(11) **EP 4 098 187 B1**

(12) **EUROPEAN PATENT SPECIFICATION**

(45) Date of publication and mention
of the grant of the patent:
**26.11.2025 Bulletin 2025/48**

(21) Application number: **20917143.8**

(22) Date of filing: **22.12.2020**

(51) International Patent Classification (IPC):
*A61B 5/11* (2006.01)    *A61B 5/00* (2006.01)
*G16H 20/30* (2018.01)    *G16H 40/63* (2018.01)
*G16H 50/20* (2018.01)

(52) Cooperative Patent Classification (CPC):
**A61B 5/6815; A61B 5/112; A61B 5/1121;
A61B 5/4023; A61B 5/6803; A61B 5/6823;
A61B 5/7246; G16H 20/30; G16H 40/63;
G16H 50/20;** A61B 5/0022; A61B 2562/0219

(86) International application number:
**PCT/KR2020/018900**

(87) International publication number:
**WO 2021/153910 (05.08.2021 Gazette 2021/31)**

(54) **METHOD AND DEVICE FOR EVALUATING BODY FUNCTION THROUGH HEAD PART MOTION ACCELERATION SENSOR**

VERFAHREN UND VORRICHTUNG ZUR AUSWERTUNG DER KÖRPERFUNKTION DURCH EINEN KOPFTEILBEWEGUNGSBESCHLEUNIGUNGSSENSOR

PROCÉDÉ ET DISPOSITIF POUR ÉVALUER UNE FONCTION CORPORELLE PAR L'INTERMÉDIAIRE D'UN CAPTEUR D'ACCÉLÉRATION DE MOUVEMENT DE PARTIE TÊTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.01.2020 KR 20200011661**

(43) Date of publication of application:
**07.12.2022 Bulletin 2022/49**

(73) Proprietor: **Beflex Inc.
Daejeon 34104 (KR)**

(72) Inventor: **JUNG, Changkeun
Daejeon 35214 (KR)**

(74) Representative: **Gulde & Partner
Patent- und Rechtsanwaltskanzlei mbB
Berliner Freiheit 2
10785 Berlin (DE)**

(56) References cited:
JP-A- 2005 172 625    KR-A- 20140 070 064
KR-A- 20190 015 851    KR-A- 20190 098 635
KR-A- 20190 115 978    US-A1- 2006 251 334
US-A1- 2017 258 370    US-A1- 2021 052 230

## Description

### Technical Field

The present invention relates to a physical performance assessment method and device through a motion acceleration sensor attached to the head, and more specifically, to a physical performance assessment method and device through a motion acceleration sensor attached to the head that is capable of assessing a patient's physical performance through the motion acceleration sensor attached to the patient's head, thereby improving the degree of accuracy in analyzing his or her physical performance.

### Background Art

As aging societies have been developed fast all over the world, older people who have medical/surgical treatments due to their aging have been drastically increased. Aging changes the structures of the cells and tissues of all of organs of the body and decreases their functions. For example, aging has a relation with sarcopenia and low physical strength, which makes a quality of life deteriorated.

To overcome such aging problems, many kinds of medical devices, treatment agents, and programs for improving the physical performance of older patients have been studied and developed.

So as to appropriately assess the physical performance of an older patient, it is important to accurately determine a degree of the patient's frailty.

So as to determine a degree of frailty, in conventional practices, short physical performance battery (SPPB) is used to measure the patient's gait speed, chair stand, and balance and thus determine his or her physical performance.

However, professionally trained personnel (hereinafter, referred to as medical staff) is needed in executing comprehensive geriatric assessment for older patients, and further, a lot of time is disadvantageously required for the assessment. Besides, the patient's physical performance is measured with the naked eye of the medical staff, and accordingly, low accuracy in the assessment may be made. Further, the assessment results may be varied according to the patient's mood or condition upon the test, and the frailty of the patient is assessed through indirect data, thereby making the degree of accuracy in the assessment results diminished.

Therefore, there is a need to develop a physical performance assessment method for calculating test data with accuracy, irrespective of surrounding situations.

KR 2019 0098635 A, KR 2019 0015851 A, KR 2019 0115978 A and US 2017/258370 A1 disclose conventional physical performance assessment methods using a conventional physical performance assessment device.

### Disclosure

### Technical Problem

Accordingly, the present invention has been made in view of the above-mentioned problems occurring in the related art, and it is an object of the present invention to provide a physical performance assessment method and device through a motion acceleration sensor attached to a patient's head that is capable of effectively improving the degree of accuracy in measuring the patient's motions.

### Technical Solution

The invention is defined by the appended set of claims. The description that follows is subjected to this limitation. Any disclosure lying outside the scope of said claims is only intended for illustrative as well as comparative purposes.

To accomplish the above-mentioned objects, according to one aspect of the present invention, there is provided a physical performance assessment method according to claim 1.

According to the present invention, the physical performance assessment method may further include the step of determining, if the protocol for the balance is executed, whether a balance test is completed based on the magnitude of the total acceleration signals with respect to a preset threshold value.

According to the present invention, if the magnitude of the total acceleration signals is greater than the preset threshold value, it is determined that the test fails or the patient quits in the middle of the test, the time taken is recorded up to the time point where the magnitude of the total acceleration signals is greater than the threshold value, and scores are applied to the recorded time according to preset score ranges.

According to the present invention, the magnitude of the total acceleration signals is an amplitude difference between a maximum peak value and a minimum peak value in the total acceleration signals.

According to the present invention, the physical performance assessment method may further include the steps of: detecting the patient's gait time points based on the gait parameters; sensing a gait start point and a gait finish point based on the gait time points; and assessing the patient's gait speed based on the time taken from the gait start point and the gait finish point.

According to the present invention, while the protocol for the chair stand is executed, if the minimum peak value is sensed earlier than the maximum peak value in the up and down acceleration and the total motion acceleration signals so that a difference between the minimum peak value and the maximum peak value is greater than the preset threshold value, it is determined that a stand-to-sit motion is performed.

According to the present invention, while the protocol for the chair stand is executed, if the maximum peak value is sensed earlier than the minimum peak value in the up and down acceleration and the total motion acceleration signals so that a difference between the minimum peak value and the maximum peak value is greater than the preset threshold value, it is determined that a sit-to-stand motion is performed.

According to the present invention, the stand parameters comprise stand-to-sit parameters for the stand-to-sit motion and sit-to-stand parameters for the sit-to-stand motion.

According to the present invention, the gait parameters further comprise vertical ground reaction forces and peak values and trough values of the vertical ground reaction forces.

According to the present invention, the gait parameters further comprise low frequency power in low frequency band and high frequency power in high frequency band, and a rate of the low frequency power with respect to the high frequency power is computed.

In an example not according to the present invention, if the constant value is less than a preset constant value capable of determining the patient's gait motion, it is determined as a normal state, and if the constant value is greater than the preset constant value, it is determined that the patient's physical performance stability becomes deteriorated.

To accomplish the above-mentioned objects, according to another aspect of the present invention, there is provided a physical performance assessment device including: a motion measurement module for collecting a patient's motion acceleration signals for the patient's motion analysis through a motion acceleration sensor attached to the patient's head; and a motion analysis module for deriving a plurality of motion parameters based on the collected motion acceleration signals, wherein a protocol for balance, a protocol for gait speed, and a protocol for chair stand are executed, wherein the motion analysis module collects, when the protocol for balance among the plurality of motion parameters is executed, the patient's three-axis motion acceleration signals for side-by-side stance, tandem stance, and semi-tandem stance to detect balance parameters capable of assessing the patient's balance performance from total acceleration signals where the three-axis motion acceleration signals are added, detects, when the protocol for gait speed among the plurality of motion parameters is executed, single stance and double stance from the motion acceleration signals to detect gait parameters from the detected results, and detects, when the protocol for chair stand among the plurality of motion parameters is executed, a minimum peak value and a maximum peak value from the up and down acceleration and the total motion acceleration signals to detect stand parameters through the relation between the minimum peak value and the maximum peak value.

Objects, characteristics and advantages of the present invention will be more clearly understood from the detailed description as will be described below and the attached drawings.

## Advantageous Effects

According to the present invention, the physical performance assessment method and device can divide the patient's motions based on the patient's motion acceleration signals collected from the motion acceleration sensor attached to the patient's head and assess the patient's physical performance, thereby minimizing an error rate.

Additionally, the physical performance assessment method and device is configured to distinguish the gait time points for the left and right legs from each other, based on the motion acceleration collected from the motion acceleration sensor attached to the patient's head, thereby increasing the conveniences of the assessment.

Further, the physical performance assessment method and device is configured to collect the up and down acceleration, the front and back acceleration, and/or the left and right acceleration so that the patient's motions can be completely analyzed to accurately detect his or her gait abnormality due to a disease.

The effectiveness of the invention is not limited as mentioned above, and it should be understood to those skilled in the art that the effectiveness of the invention may include another effectiveness as not mentioned above from the detailed description of the present invention.

## Brief Description of Drawings

FIGs. 1a and 1b are schematic views showing a physical performance assessment device through a motion acceleration sensor attached to the head according to the present invention.

FIG. 2 is a block diagram showing the physical performance assessment device according to the present invention.

FIG. 3 is a flowchart showing a physical performance assessment method through a motion acceleration sensor attached to the head according to the present invention.

FIG. 4 is a schematic diagram showing a balance test protocol according to the present invention.

FIG. 5a is an exemplary view showing balance parameters derived by axis according to the present invention.

FIG. 5b is an exemplary view showing the test parameters based on the balance test according to the present invention, wherein the test fails.

FIG. 5c is an exemplary view showing the test parameters based on the balance test according to the present invention, wherein the test is completed.

FIG. 6 is a schematic diagram showing a gait speed test protocol according to the present invention.

FIG. 7 is a graph showing motion acceleration by time according to the present invention.

FIG. 8 is an exemplary view showing a process of collecting gait acceleration signals to detect gait time points according to the present invention.

FIG. 9 is a flowchart showing a gait parameter detection method according to the present invention.

FIG. 10 is a graph showing the power by frequency for three axes according to the present invention.

FIG. 11 is a schematic diagram showing a chair stand protocol according to the present invention.

FIG. 12 is a graph showing motion acceleration by time according to the present invention.

FIG. 13 is an exemplary view showing a stand-to-sit motion according to the present invention, which corresponds to an area A of FIG. 12.

FIG. 14 is an exemplary view showing a sit-to-stand motion according to the present invention, which corresponds to an area B of FIG. 12.

**Best Mode for Invention**

Embodiments of the present invention just provide the principle of the present invention. Therefore, the implementation of the principle of the present invention and various devices included in the concept and scope of the present invention may be carried out by a person having ordinary skill in the art. Further, it should be appreciated that all terms and embodiments described in the present invention are obviously intended to allow the concept of the present invention to be understood and are not limited by the disclosed embodiments of the present invention.

In the description, terms, such as the first, the second, and the like may be used to describe various elements that are in the same situation, independently of each other, and it should understood that no main/sub or master/slave positions are given to the elements.

Some or all of the features of various embodiments of the present invention may be combined to one another, and they may technically operate cooperatively with one another, which will be understood by a person having ordinary skill in the art. Further, the respective embodiments of the present invention may be carried out independently of each other or combinedly with each other.

Hereinafter, exemplary embodiments of the present invention will be described in detail below with reference to the accompanying drawings.

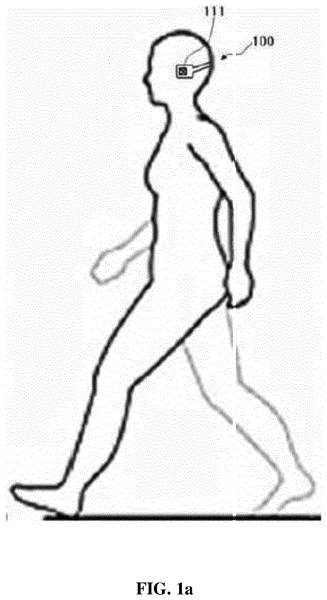

Now, an explanation of a configuration of a physical performance assessment device 100 according to the present invention will be given with reference to FIGs. 1a to 2.

**Explanation of physical performance assessment device**

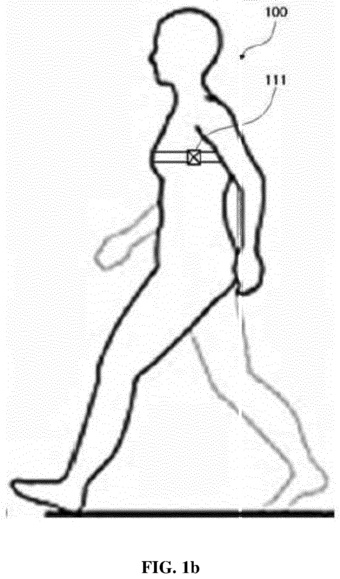

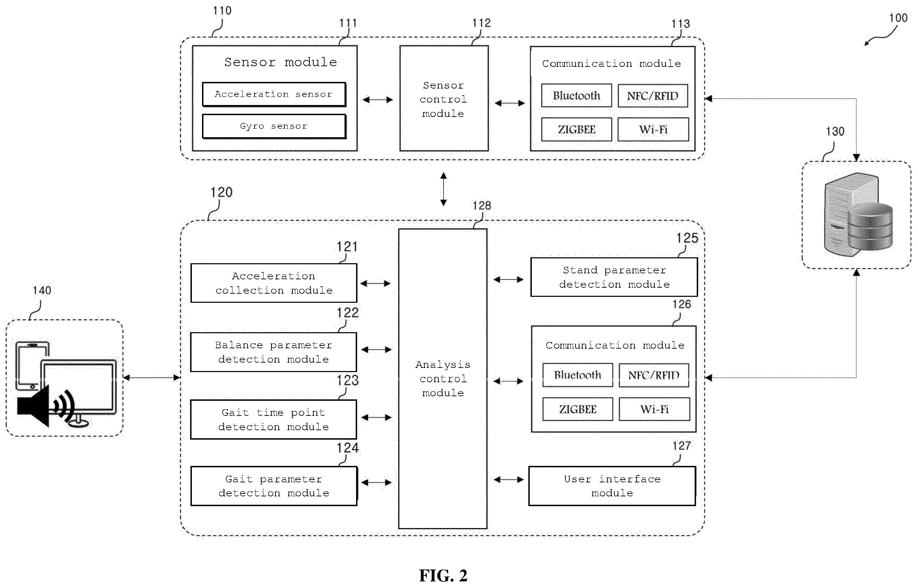

FIGs. 1a and 1b are schematic views showing a physical performance assessment device through a motion acceleration sensor attached to the head according to the present invention. FIG. 2 is a block diagram showing the physical performance assessment device according to the present invention.

Referring to FIGs. 1a and 1b, the physical performance assessment device 100 according to the present invention serves to assess the physical performance of older patients based on objective assessment metrics such as balance, gait speed, and chair stand. That is, the physical performance assessment device 100 is defined as a device for executing a short physical performance battery (SPPB) protocol. In this case, the SPPB protocol is an assessment tool (or a measurement protocol) that combine the results of three objective assessment metrics, that is, balance, gait speed, and chair stand, which are usefully and easily measured among the physical performance assessment methods for older patients. According to the present invention, the SPPB protocol can be called 'physical performance assessment tool', for the conveniences of the description. An explanation of the physical performance assessment method using the physical performance assessment tool will be given later.

The physical performance assessment device 100 may be a wearable device having a motion acceleration sensor attached to a headset, an earphone, a cap, or the like, which is worn on a patient's head. As shown in FIG. 1a, the physical performance assessment device 100 is attached to the headset. However, of course, the physical performance assessment device 100 may be worn on the patient's waist or upper body around the chest (See FIG. 1b), without being limited thereto.

Referring to FIG. 2, the physical performance assessment device 100 includes a motion measurement module 110, a motion analysis module 120, a database 130, and a notification module 140.

The motion measurement module 110 serves to measure the patient's motion acceleration signals using the motion acceleration sensor attached to his or her head. In this case, the motion acceleration signals may be up and down motion acceleration $az$, left and right motion acceleration $ax$, and front and back motion acceleration $ay$.

Referring to FIG. 1a, the motion measurement module 110 is attached to the headset. However, the motion measurement module 110 may be detachably attached to the patient's upper body around the chest through a band (See FIG. 1b). Accordingly, the motion measurement module 110 may be attached to the upper body as well as the head. For example, the motion measurement module 110 may be attached to the patient's upper arm, ear, head, or shoulder through a band or the like. However, if the motion measurement module 110 is detachably attached to the body region under the upper body, the degree of accuracy in measurement may be diminished, and accordingly, the motion measurement module 110 is desirably attached to the body region close to the head, excepting the lower body. According to the present invention, further, the motion measurement module 110 may be accommodated in a separate housing from the motion analysis module 120 or in the same housing as the motion analysis module 120.

Referring to FIG. 2, the motion measurement module 110 includes a sensor module 111, a sensor control module 112, and a communication module 113.

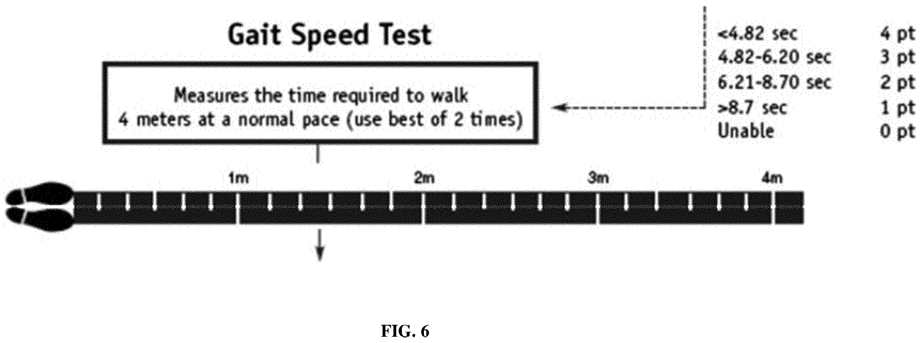

The sensor module 111 serves to measure the sensing values (hereinafter, referred to as acceleration signals or sensor values) required for the motion analysis of the motion analysis module 120 and transmit the measured values to the motion analysis module 120. According to the present invention, sensing the sensor values required for gait analysis through the sensor module 111 will be described, but the sensor module 111 collects the sensed motion acceleration, detects gait time points and gait parameters from the collected motion acceleration, and transmits the detected results to the motion analysis module 120. Further, the sensor module 111 includes a motion acceleration sensor and a gyro sensor, and of course, the sensor module 111 may measure the sensing values through a GPS sensor. A process of detecting the gait time points and the gait parameters will be explained later with reference to FIG. 6.

The sensor control module 112 serves to control all operations of the motion measurement module 110.

The communication module 113 serves to transmit the sensor values sensed by the sensor module 111 to the motion analysis module 120. For example, the communication module 113 includes Bluetooth, Near Field Communication (NFC), Radio-Frequency Identification (RFID), Zigbee module, Wi-Fi, and the like.

Referring to FIG. 2, the motion analysis module 120 serves to analyze physical performance analysis information based on the motion measurement module 110. The physical performance analysis information is defined as information of the patient's gait speed, balance, and stand-to-sit and sit-to-stand performance analyzed, based on the SPPB protocol, and it should be desirably understood that the physical performance analysis information is different from motion parameters as will be discussed later.

In specific, the motion analysis module 120 includes a motion acceleration collection module 121 for receiving the motion acceleration signals from the sensor module 111 of the motion measurement module 110 to collect motion acceleration, a balance parameter detection module 122 for deriving balance parameters from the collected motion acceleration, a gait time point detection module 123 for detecting minimum and maximum peaks of the motion acceleration from the collected motion acceleration to detect gait time points, a gait parameter detection module 124 for deriving gait parameters based on the detected gait time points, and a stand parameter detection module 125 for sensing stand-to-sit and sit-to-stand motions from the collected motion acceleration to detect stand-to-sit parameters and sit-to-stand parameters.

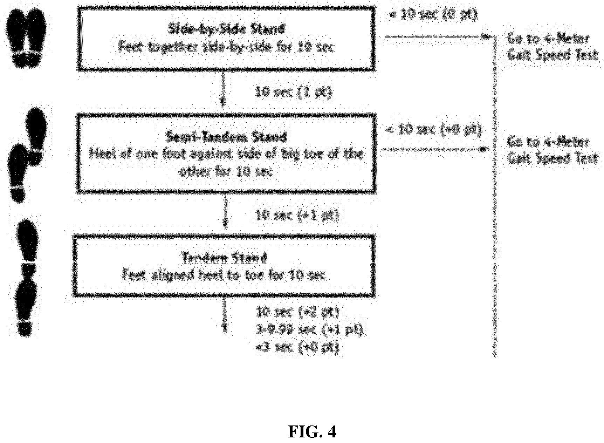

Moreover, the motion analysis module 120 may further include a communication module 126 for transmitting signals to the motion measurement module 110 and the notification module 140 of the physical performance assessment device 100. The specific operations of the gait time point detection module 123 and the gait parameter detection module 124 will be explained later with reference to FIGs. 4 to 14.

If the motion acceleration collection module 121 is provided integrally with the motion measurement module 110, the communication module 113 of the motion measurement module 110 is connected directly to the motion analysis module 120 to transmit the collected motion acceleration signals to the motion analysis module 120. Further, the gait analysis information is outputted through the notification module 140. For example, the gait analysis information may be outputted through a speaker, a smartphone, a computer, wireless earphones, and the like.

The notification module 140 serves to convert test guiding information for the SPPB protocol for assessing the patient's physical performance through the detection of various parameters using the motion analysis module 120 into the form recognized by the patient, such as sound or video and output the converted information. For example, if a balance test is performed, the notification module 140 produces a sound, "Please stand side-by-side on a floor" through the speaker provided in the physical performance assessment device 100 or the smartphone, computer, or wireless earphones connected to the physical performance assessment device 100, or displays the notification information through a display module.

Further, the notification module 140 serves to convert the physical performance analysis information produced from the motion analysis module 120 into the information recognized by the patient, such as sound or video, and output the converted information. For example, if there is a need to correct a step length, a sound, "Please reduce a step length" is outputted through the speaker or the smartphone, computer, or wireless earphones, and otherwise, an alarm sound such as a beep sound may be outputted. In this case, if a step length to be corrected is long, the alarm sound is outputted at a fast speed, and contrarily, if a step length to be corrected is short, the alarm sound is outputted at a slow speed. Through the alarm sound, accordingly, the patient can recognize the step length to be corrected and correct his or her step length. In specific, the notification module 140 includes the wireless earphones or the speaker, but according to the present invention, basically, the motion acceleration sensor is attached to the earphones worn on the patient's head, so that the physical performance analysis information can be transmitted directly to the patient's ear through the physical performance assessment device 100. Further, the notification module 140 is connected to the smartphone, computer, dedicated display, or the like to output the correction information to the form of the video, and accordingly, the correction information may be outputted to various forms.

Further, the physical performance assessment device 100 transmits the physical performance analysis information derived by the motion analysis module 120 to the database 130. In this case, the physical performance analysis information is accumulatedly stored so that the gait positions of the patient can be checked according to time evolution.

If large pieces of physical performance analysis information are accumulatedly stored, the stored data is utilized as big data that can be used for all kinds of statistical analysis. The physical performance assessment device 100 has an analysis control module 128 for controlling all operations of the motion analysis module 120.

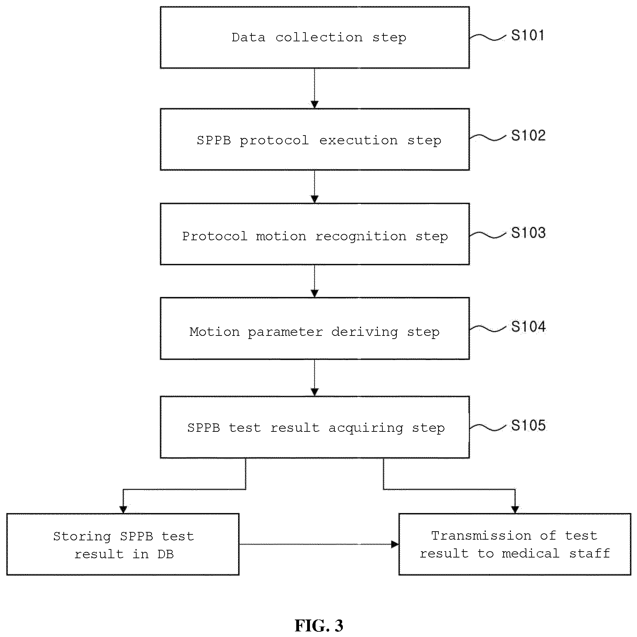

Hereinafter, an explanation of a physical performance assessment method using the SPPB protocol according to the present invention will be given with reference to FIG. 3.

FIG. 3 is a flowchart showing a physical performance assessment method according to the present invention.

Referring to FIG. 3, the motion measurement module 110 collects data from the physical performance assessment device 100 attached to the patient's head (at step S101).

Next, the motion analysis module 120 executes the SPPB protocol (at step S102). Through the SPPB protocol, the three assessment metrics as mentioned above, that is, balance, gait speed, and chair stand are executed, and an explanation of the SPPB protocol will be given in detail later with reference to FIGs. 4 to 14.

Next, the motion analysis module 120 recognizes the patient's motions by protocol (at step S103) and derives motion parameters (at step S104). In this case, the motion parameters include balance parameters, gait parameters, and stand parameters as a plurality of metrics derived from the SPPB protocol. In this case, each parameter includes at least one metric.

If the balance test is carried out, the protocol motions for three positions of the patient, that is, side-by-side stance, tandem stance, and semi-tandem stance are recognized, and based on the recognized positions, the balance parameters can be derived. In this case, the side-by-side stance is a position where the upper limbs come into contact with the trunk in an upright standing position with the legs open to a shoulder width, the semi-tandem stance is a position where one foot is placed slightly further ahead the other foot, and the tandem stance is a position where one foot is placed directly in front of the other foot, with the toes of one foot almost touching the heel of the other foot. An explanation of the balance test will be given in detail later with reference to FIGs. 4 to 5c.

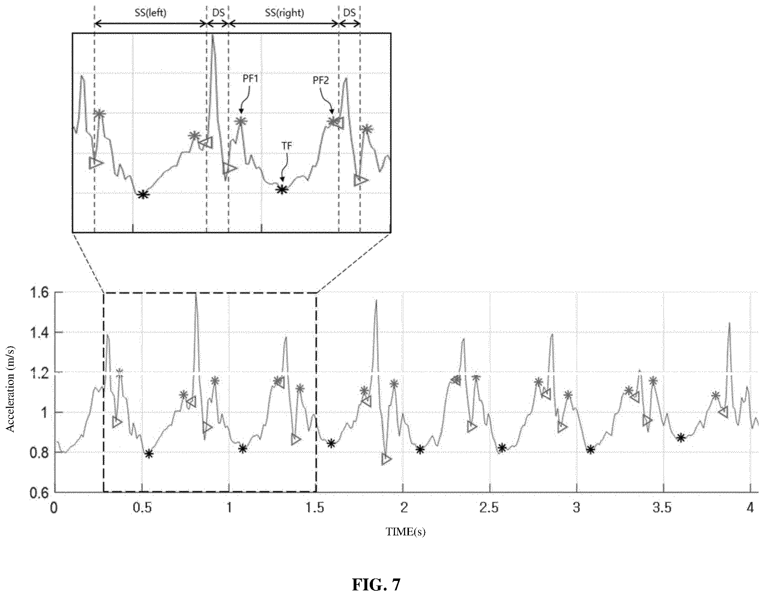

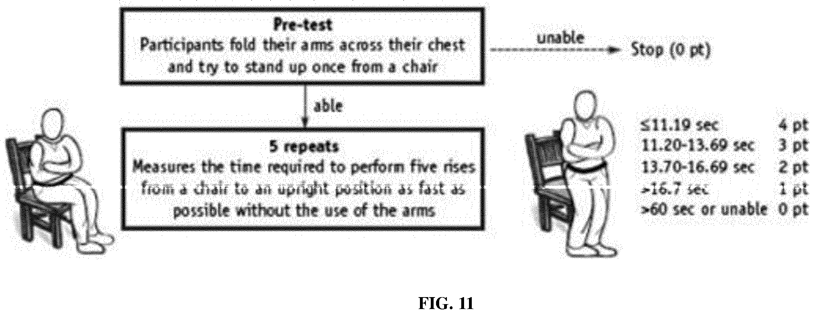

If the gait test is carried out, the patient's gait time points are detected based on his or her motion acceleration to recognize the gait motion including the gait peed of the patient, and based on the recognized motion, the gait parameters can be derived. The gait parameters include detection components for analyzing the gait motion of the patient based on the detected gait time points, for example, double support (DS), single support (SS), cadence, and step width. An explanation of the patient's gait parameters will be given in detail later with reference to FIG. 7. If stand-to-sit and sit-to-stand tests are carried out, further, stand-to-sit and sit-to-stand motions are recognized based on the patient's up and down acceleration or the analysis value of the signal vector magnitude, and based on the recognized motions, a plurality of stand parameters can be derived. An explanation of the stand-to-sit and sit-to-stand tests will be given in detail later with reference to FIGs. 11 to 14.

Next, the SPPB test results are acquired (at step S105), and the obtained results are stored in the database DB or transferred to the medical staff. In this case, after the acquired results are stored in the database DB, they may be transferred to the medical staff through a wired or wireless network.

Now, an explanation of a process of executing the SPPB protocol for the balance test according to the present invention will be given in detail with reference to FIGs. 4 to 5c.

### SPPB protocol for balance test

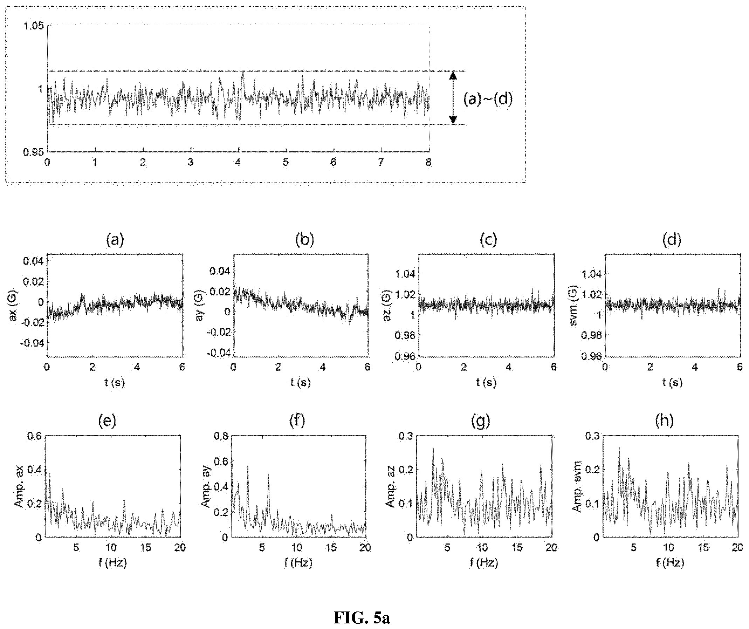

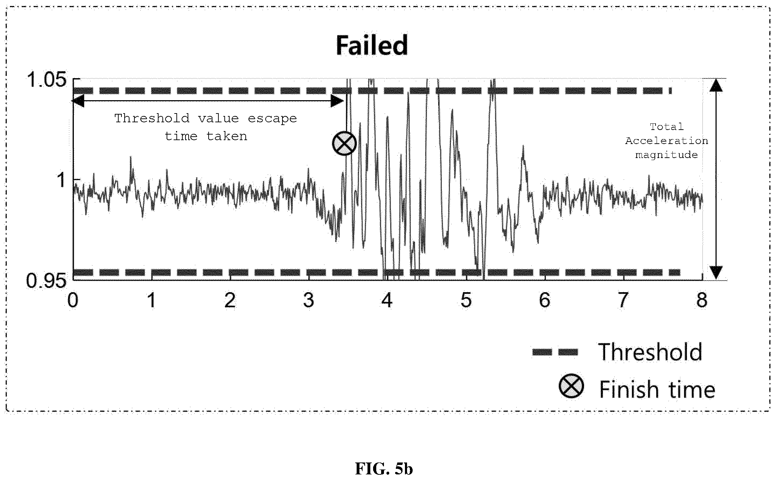

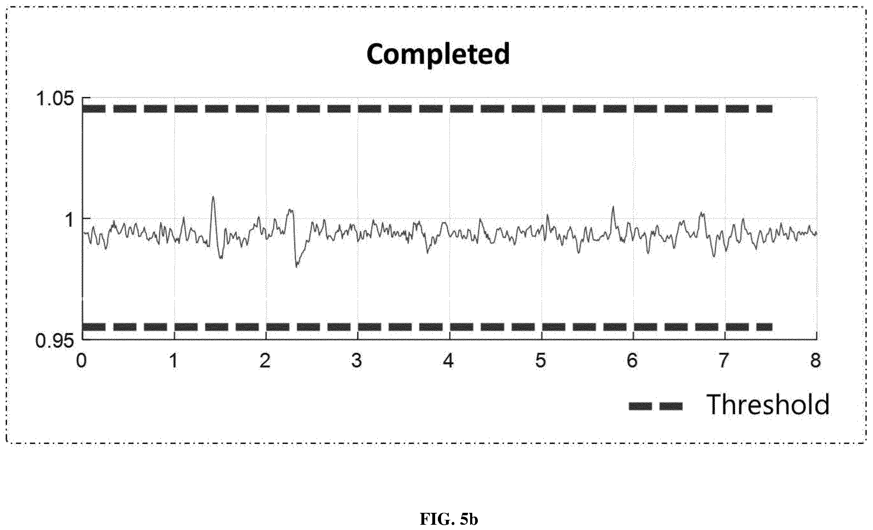

FIG. 4 is a schematic diagram showing a balance test protocol according to the present invention. FIG. 5a is an exemplary view showing the balance parameters derived by axis according to the present invention. FIG. 5b is an exemplary view showing the test parameters based on the balance test according to the present invention, wherein the test fails. FIG. 5c is an exemplary view showing the test parameters based on the balance test according to the present invention, wherein the test is completed. FIGs. 5b and 5c show total acceleration signals (hereinafter referred to as "motion acceleration signals") that are obtained by adding the acceleration signals by axis.

The balance test is carried out by repeatedly executing the SPPB protocol for the three positions. That is, the motion parameters for the side-by-side stance, the semi-tandem stance, and the tandem stance are derived, whether each position is held for 10 seconds is assessed, and scores are applied to the assessed results, which are repeatedly executed.

In specific, the test is started through the patient's input. In this case, the patient's input is performed by the smartphone or PC connected to the physical performance assessment device 100 or by a button additionally included in the physical performance assessment device 100. In this case, an explanation of the test protocol will be given to the patient. For example, the explanation of the test protocol is outputted to the form of sound through the speaker module included in the physical performance assessment device 100 or to the form of text, image, video, and the like through the smartphone or PC connected to the physical performance assessment device 100.

Further, as shown in FIG. 4, the assessment is carried out in the order of the side-by-side stance, the semi-tandem stance, and the tandem stance, but if necessary, it may be changed in order. When the position is changed, the patient goes to 4-meter so that the gait speed test can be carried out together. An explanation of the gait speed test will be given later.

Referring to FIG. 5a, the patient's motion acceleration signals on the respective axes ax, ay, and az are collected in the order of assessment as mentioned above. For example, the left and right acceleration signals ax (See the graph (a) of FIG. 5a), the up and down acceleration signals az (See the graph (b) of FIG. 5a), and the front and back acceleration signals ay (See the graph (c) of FIG. 5a) are collected. In this case, the balance parameters are detected from the motion acceleration signals through the balance parameter detection module 122, and the detectable balance parameters are listed in Table 1.

<Table 1>

|  | Parameter | Description |
|---|---|---|
| (a) | ax amplitude | Difference between max and min of ax signals |
| (b) | ay amplitude | Difference between max and min of ay signals |
| (c) | az amplitude | Difference between max and min of az signals |
| (d) | svm amplitude | Difference between max and min of svm signals |
| (e) | ax high frequency power | Power sum of signals having 0.5 Hz or more |
| (f) | ay high frequency power | Power sum of signals having 0.5 Hz or more |
| (g) | az high frequency power | Power sum of signals having 0.5 Hz or more |
| (h) | svm high frequency power | Power sum of signals having 0.5 Hz or more |

Referring to a graph (d) of FIG. 5a, further, if the collected three axis acceleration signals ax, ay and az are added, the total motion acceleration magnitude (Signal Vector Magnitude (SVM)) is calculated. For example, it can be appreciated that the total motion acceleration magnitude is about 0.4. In this case, the total motion acceleration magnitude is calculated by the following mathematical expression 1.

<Mathematical expression 1>

$$|\mathbf{v}| = \sqrt{x^2 + y^2 + z^2}$$

In this case, as shown in graphs (e) to (h) of FIG. 5a, the frequencies of the signals by axis are analyzed to thus analyze shaking in the positions. Generally, in the standing position on both feet, if shaking of 0.1 Hz or less occurs, it means that stable position is held, and if shaking is in the range of 0.1 to 0.5 Hz, it means that the vestibular organ is abnormal. Further, if shaking is in the range of 0.5 to 1 Hz, it means that somatic senses from the lower limb and spine are abnormal, and if shaking of 1 Hz or more occurs, the central nervous system is abnormal. So as to measure the balance abnormality of older patients, accordingly, the acceleration signals by axis for the frequencies f(Hz) of 0.5 Hz or more and the total acceleration magnitude as the sum of the acceleration signals are collected, as shown in the graphs (e) to (h) of FIG. 5a. As a result, the total motion acceleration magnitude can be recognized.

After that, as shown in FIGs. 5b and 5c, it is determined whether the test is completed or fails based on the acceleration magnitude with respect to preset threshold values. In this case, the preset threshold values represent the amplitude of the acceleration signals preset by the patient, and in FIGs. 5b and 5c, the preset threshold values are about 0.1. However, of course, the preset threshold values may be changed according to embodiments of the present invention.

In specific, referring to FIG. 5b, if an amplitude difference between a maximum peak and a minimum peak of the total acceleration signals is greater than the preset threshold values, it is determined that the test fails or the patient quits in the middle of the test, and accordingly, the time taken is recorded up to the time point where the amplitude difference is over the threshold values to provide scores. In this case, if the amplitude difference is less than the threshold values, it is determined that the test is completed, and accordingly, the test is finished. That is, if the amplitude difference is less than the threshold values, it is desirably determined that the patient's motions are stable.

Further, if scores are applied to the total acceleration signals collected according to the three positions, they are applied according to preset score ranges. According to the preset score ranges, if the side-by-side and semi-tandem positions are held for 10 seconds or more, one point (1 pt) is applied to each position, and if the tandem position is held for three seconds or more, one point (1 pt) is applied. Further, if the tandem position is held for 10 seconds or more, two points (2 pt) are applied, and the maximum score is set to four points. In this case, desirably, the preset score ranges are generally based on the SPPB used to determine the frailty of the older patient.

Accordingly, the motion analysis module 120 extracts the shaking in the three axis positions through the balance parameters detected from the balance parameter detection module 122, and the scores are applied according to the patient's position holding degrees for given time, thereby assessing the balance performance of the patient.

Now, an explanation of the gait speed test according to the present invention will be given in detail with reference to FIGs. 6 to 10.

## SPPB protocol for gait speed test

FIG. 6 is a schematic diagram showing a gait speed test protocol according to the present invention. FIG. 7 is a graph showing motion acceleration by time according to the present invention.

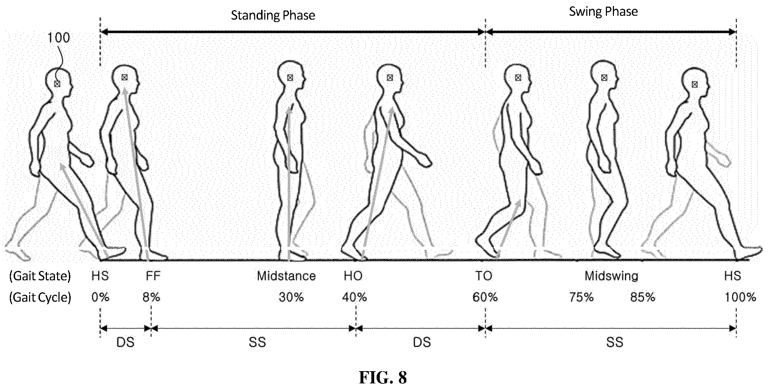

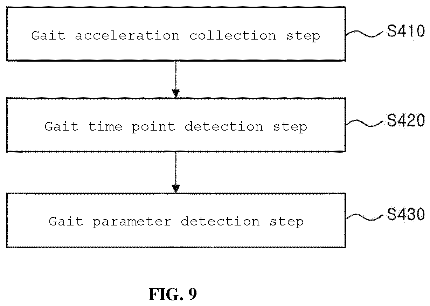

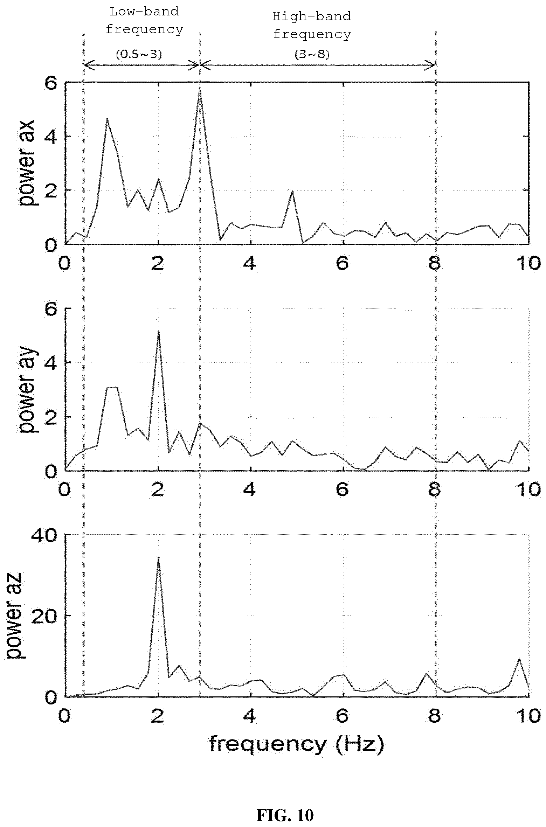

FIG. 8 is an exemplary view showing a process of collecting gait acceleration signals to detect gait time points according to the present invention. FIG. 9 is a flowchart showing a gait parameter detection method according to the present invention. FIG. 10 is a graph showing the power by frequency for three axes according to the present invention. Referring first to FIG. 7, a horizontal axis represents time s, and a vertical shaft acceleration $m/s^2$. When the gait speed test is carried out, the process of notifying test start and test explanation is the same as in the above-mentioned balance test, and therefore, an explanation of the process will be avoided.

Referring to FIG. 6, the gait speed test is carried out by measuring the time (sec) taken to walk 4 meters in a standing position at a normal pace. Accordingly, the patient's gait start and finish points are sensed to record the time taken, and based on the time taken, scores are applied. For example, if the test fails, a zero point is applied, and if the time taken is greater than 8.7 seconds, one point is applied. If the time taken is in the range of 6.21 to 8.7 seconds, two points are applied, if the time taken is in the range of 4.82 to 6.20 seconds, three points, and if the time taken is less than 4.82 seconds, four points.

For example, if it is assumed that there is a patient walks 4 meters within four seconds, the gait acceleration signals of the patient are collected (at step S910 of FIG. 9), the gait time points are detected from the collected gait acceleration signals through the gait time point detection module 123 (at step S920), and the gait parameters are detected through the gait parameter detection module 122 (at step S930). In this case, the gait parameters related to the detectable gait time points are listed in Table 2.

<Table 2>

| | | Parameter | Description |
|---|---|---|---|
| | (a) | Single stance time | Time during which single stance is maintained |
| | (b) | Single stance time left | Time during which single stance (left foot) is maintained |
| | (c) | Single stance time right | Time during which single stance (right foot) is maintained |
| | (d) | Double stance time | Time during which double stance is maintained |
| | (e) | Double stance time left | Time during which double stance is maintained (after single left foot stance) |
| | (f) | Double stance time right | Time during which double stance is maintained (after single right foot stance) |
| | (g) | Single stance time variance | Variance for time during which single stance is maintained |

(continued)

| | Parameter | Description |
|---|---|---|
| (h) | Single stance time left variance | Variance for time during which single stance (left foot) is maintained |
| (i) | Single stance time right variance | Variance for time during which single stance (right foot) is maintained |
| (j) | Double stance time variance | Variance for time during which double stance is maintained |
| (k) | Double stance time left variance | Variance for time during which double stance is maintained (after single left foot stance) |
| (l) | Double stance time right variance | Variance for time during which double stance is maintained (after single right foot stance) |

In specific, referring to FIG. 7, the patient's motion acceleration signals from a landing time point ▷ to the intermediate time point ◁ of a ground reaction force peak are defined as single stance SS. In this case, the single stance includes the single stance on the left foot SS(left) and the single stance on the right foot SS(right), and the respective single stances are periodically repeated. Further, the rest stance excepting the single stance is defined as double stance DS.

Like this, the total acceleration signals including the single stance SS and the double stance DS are defined as a gait cycle. As shown in FIG. 8, the gait cycle is divided into a standing phase and a swing phase, and during one gait cycle, the single stance SS and the double stance DS appear two times, respectively. Further, if the gait speed of the patient becomes faster, the single stance SS becomes increased, and the double stance SS becomes decreased. Also, the standing phase includes five stages such as heel strike (HS), foot flat (FF), midstance, heel off **(HO), and** toe off (TO). Further, the swing phase includes three stages such as toe off (TO), midswing, and heel strike (HS). According to the present invention, the gait stages are described with respect to the right foot, and the left foot is described as the opposite side to the right foot.

Further, the gait parameter detection module 124 detects a ground reaction force peak value and a ground reaction force trough value as the gait parameters. Referring to FIG. 7, there are three peaks * in the single stance SS, that is, between the minimum point ▷ and the maximum point ◁ of the acceleration. In this case, a light gray peak * located on one single stance SS (or located on the left side of one single stance SS) represents a first vertical ground reaction force peak value PF1, a dark gray peak * located on one single stance SS (or located on the right side of one single stance SS) represents a second vertical ground reaction force peak value PF2, and a black peak * located between the peaks located on the left and right sides of one single stance SS) represents a vertical ground reaction force trough value TF1. The gait parameters are listed in Table 3.

<Table 3>

| | Parameter | Description |
|---|---|---|
| (a) | First peak force | Peak value of first vertical ground reaction force |
| (b) | First peak force left | Peak value of first vertical ground reaction force (left foot) |
| (c) | First peak force right | Peak value of first vertical ground reaction force (right foot) |
| (d) | Trough force | Trough value of vertical ground reaction force |
| (e) | Trough force left | Trough value of vertical ground reaction force (left foot) |
| (f) | Trough force right | Trough value of vertical ground reaction force (right foot) |
| (g) | Second peak force | Peak value of second vertical ground reaction force |
| (h) | Second peak force left | Peak value of second vertical ground reaction force (left foot) |
| (i) | Second peak force right | Peak value of second vertical ground reaction force (right foot) |
| (j) | First peak force variance | Variance of peak value of first vertical ground reaction force |
| (k) | First peak force left variance | Variance of peak value of first vertical ground reaction force (left foot) |
| (l) | First peak force right variance | Variance of peak value of first vertical ground reaction force (right foot) |
| (m) | Trough force variance | Variance of trough value of vertical ground reaction force |
| (n) | Trough force left variance | Variance of trough value of vertical ground reaction force (left foot) |
| (o) | Trough force right variance | Variance of trough value of vertical ground reaction force (right foot) |

(continued)

| | Parameter | Description |
|---|---|---|
| (p) | Second peak force variance | Variance of peak value of second vertical ground reaction force |
| (q) | Second peak force left variance | Variance of peak value of second vertical ground reaction force (left foot) |
| (r) | Second peak force right variance | Variance of peak value of second vertical ground reaction force (right foot) |

Further, the gait parameter detection module 124 detects the power by frequency band with respect to the three-axis acceleration ax, ay, and az as the gait parameters. The gait parameters are listed in Table 4.

<Table 4>

| | Parameter | Description |
|---|---|---|
| (a) | ax low frequency power | Low frequency power (0.5 to 3 Hz) |
| (b) | ax high frequency power | High frequency power (3 to 8 Hz) |
| (c) | ay low frequency power | Low frequency power (0.5 to 3 Hz) |
| (d) | ay high frequency power | High frequency power (3 to 8 Hz) |
| (e) | az low frequency power | Low frequency power (0.5 to 3 Hz) |
| (f) | az high frequency power | High frequency power (3 to 8 Hz) |

In specific, as shown in FIG. 10, it can be appreciated that the power at low frequency band (in the range of 0.5 to 3 Hz) is higher than that at high frequency band (in the range of 3 to 8 Hz) according to the power by frequency band (power ax, power ay, and power az) with respect to the three-axis acceleration (ax, ay, and az). In this case, the motions at the low frequency band are defined as the frequencies which have a high probability that the motions are intended by a person, and the motions at the high frequency band are defined as the frequencies caused by the motions not intended by a person, for example, shaking.

In this case, a rate of the low frequency band power with respect to the high frequency band power is calculated by the following Mathematical expression 2 and obtained as a constant value. In this case, if the constant value is less than a preset constant value, it is determined as a normal state, and if greater than the preset constant value, it is determined as an abnormal state.

<Mathematical expression 2>

$$\frac{\text{High-band frequency (square of total power in the 3-8Hz band)}}{\text{Low-band frequency (square of total power in the 0.5-3Hz band)}} = \text{Constant}$$

In this case, the preset constant value is 0.5, and if the constant value obtained through the Mathematical expression 2 is less than 0.5, the normal state is determined. However, if greater than 0.5, it is possible to have neurological diseases (for example, Parkinson's disease).

Referring to the power by frequency band (power ax) with respect to the left and right motion acceleration of FIG. 10, the power at low frequency band (in the range of 0.5 to 3 Hz) is present in the range of about 1 to 6, and the power at high frequency band (in the range of 3 to 8 Hz) is present in the range of about 0 to 2. As mentioned above, that is, the rate of the low frequency band power with respect to the high frequency band power is less than 0.5, it can be determined as a normal state.

Further, the gait parameter detection module 124 further detects the gait parameters as listed in Table 5. In this case, the gait parameters (a) to (f) of Table 5 are disclosed in detail in Korean Patent No. 10-2055661, which are suggested for the reference of the present invention. The vertical oscillations listed in the gait parameters (h) to (j) of Table 5 can be calculated by Mathematical expression 3.

<Table 5>

| | Parameter | Description |
|---|---|---|
| (a) | Step count | The number of steps |
| (b) | Cadence | The number of steps per minute |
| (c) | Speed | Speed |
| (d) | Step length | Step length |
| (e) | Step width | Step width |
| (f) | Head angle | Vertical inclination angle of head |
| (g) | Head angle variance | Variance of vertical inclination angle of head |
| (h) | Vertical oscillation | Vertical oscillation |
| (i) | Vertical oscillation left | Vertical oscillation (left) |
| (j) | Vertical oscillation right | Vertical oscillation (right) |

<Mathematical expression 3>

Vertical oscillation = Constant x Central frequency power oscillation

Accordingly, the motion analysis module 120 detects the gait time points from the gait parameters detected through the gait parameter detection module 124, and based on the gait time points, the start and finish points are sensed. As a result, scores are applied according to the time taken from the start point to the finish time of the gait time points, thereby assessing the patient's gait performance.

**SPPB protocol for chair stand test**

Hereinafter, an explanation of the chair stand test according to the present invention will be given with reference to FIGs. 11 to 14. The chair stand test includes tests for a stand-to-sit motion and a sit-to-stand motion.

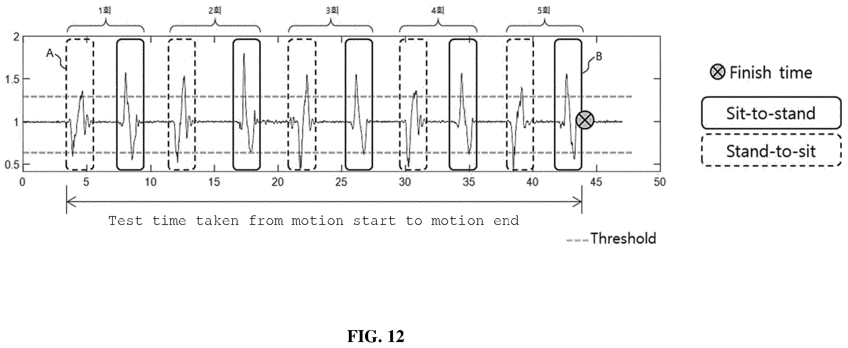

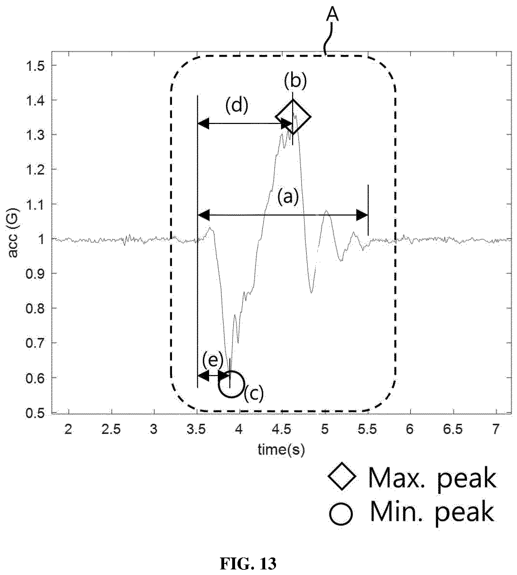

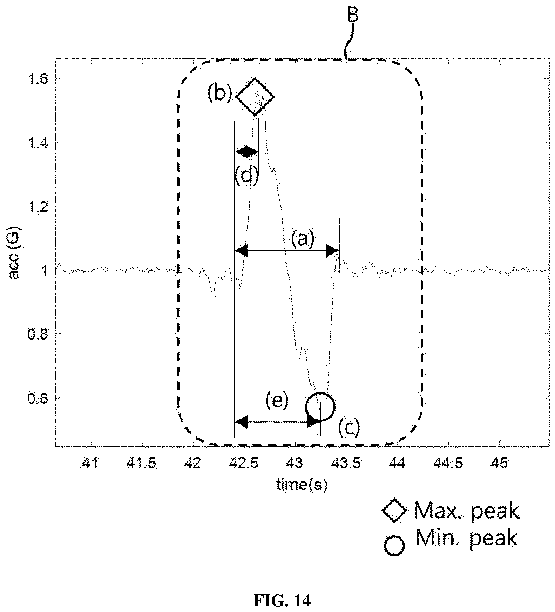

FIG. 11 is a schematic diagram showing a chair stand protocol according to the present invention. FIG. 12 is a graph showing motion acceleration by time according to the present invention. FIG. 13 is an exemplary view showing a stand-to-sit motion according to the present invention, which corresponds to an area A of FIG. 12. FIG. 14 is an exemplary view showing a sit-to-stand motion according to the present invention, which corresponds to an area B of FIG. 12. When the chair stand test is carried out, the process of notifying test start and test explanation is the same as in the above-mentioned balance test, and therefore, an explanation of the process will be avoided.

Referring to FIG. 11, the chair stand test is carried out by repeating the sit-to-stand motion five times, without the use of the patient's hands (that is, with his or her arms folded on the chest), measuring the time taken at the last point, and applying scores according to the measured time. For example, if the time taken is greater than 60 seconds or the motion is not carried out, a zero point is applied, if the time taken is greater than 16.7 seconds, one point, if the time taken is in the range between 13.7 and 16.69 seconds, two points, if the time taken is in the range between 11.2 and 13.69 seconds, three points, and if the time is less than 11.19 seconds, four points.

Further, the chair stand test is carried out by using the up and down acceleration az or the total acceleration signal magnitude as analysis values to thus sense the minimum peak value and the maximum peak value of the analysis values, so that the motions of the patient can be recognized. Referring to FIG. 12, while the sit-to-stand motion is carried out by the patient five times, the time taken is measured at the last point $\otimes$ after the motion has been repeated five times, and it can be appreciated that the time taken is for example 40 seconds. Further, the stand-to-sit motion and the sit-to-stand motion are repeatedly carried out, while having one pair of a minimum peak value (hereinafter, referred to as min value) and a maximum peak value (hereinafter, referred to as max value) in the total acceleration signals. In this case, the stand-to-sit motion and the sit-to-stand motion are performed for about 2 to 3 seconds, respectively.

In specific, if the min value occurs earlier than the max value so that the difference between the two values is greater than a preset threshold value, the stand parameter detection module 125 determines (senses) that the patient performs the stand-to-sit motion. Further, the stand parameter detection module 125 sensing the stand-to-sit motion detects stand-to-sit parameters from the motion acceleration signals. For example, the detectable stand-to-sit parameters are listed in Table 6. Referring further to FIG. 13, at the time point where the first stand-to-sit motion is performed (the area A of FIG. 13 in the range of about 3.5 to 5.5 seconds), the time (a) taken to perform the stand-to-sit motion is about 2 seconds, and the min value (c) is sensed earlier than the max value (b). In this case, a difference between the min value (c) and the max value (b) is greater than the preset threshold value, and accordingly, the motion is sensed as the stand-to-sit motion. The threshold value in FIG. 12 is arbitrarily set, and in some cases, therefore, it may be changed. Referring to FIG. 12, further, the value from the start point to the min value (c) is defined as the time (e) taken from the start to a trough value (d), and the value from the start point to the max value (b) is defined as the trough value (d).

<Table 6>

|  | | Parameter | Description |
|---|---|---|---|
| (a) | | Time stand-to-sit | Time taken |
| (b) | | Peak stand-to-sit | Peak value |
| (c) | | Time peak stand-to-sit | Time taken from start to peak value |
| (d) | | Trough stand-to-sit | Trough value |
| (e) | | Time trough stand-to-sit | Time taken from start to trough value |
| (f) | | ax low frequency power | Low frequency power (0.5 to 3 Hz) |
| (g) | | ax high frequency power | High frequency power (3 to 8 Hz) |
| (h) | | ay low frequency power | Low frequency power (0.5 to 3 Hz) |
| (i) | | ay high frequency power | High frequency power (3 to 8 Hz) |
| (j) | | az low frequency power | Low frequency power (0.5 to 3 Hz) |
| (k) | | az high frequency power | High frequency power (3 to 8 Hz) |
| (l) | | svm low frequency power | Low frequency power (0.5 to 3 Hz) |
| (m) | | svm high frequency power | High frequency power (3 to 8 Hz) |

Further, if the max value occurs earlier than the min value so that the difference between the two values is greater than a threshold value, the stand parameter detection module 125 determines (senses) that the patient performs the sit-to-stand motion. Further, the stand parameter detection module 125, which has sensed the sit-to-stand motion, detects sit-to-stand parameters from the motion acceleration signals. For example, the detectable sit-to-stand parameters are listed in Table 7. Referring further to FIG. 14, at the time point where the last stand-to-sit motion is performed (the area B of FIG.14 in the range of about 42 to 44 seconds), the time (a) taken to perform the sit-to-stand motion is about 2 seconds, and the max value (b) is sensed earlier than the min value (c). In this case, a difference between the max value (b) and the min value (c) is greater than a preset threshold value, the motion is sensed as the sit-to-stand motion. Referring to FIG. 14, further, the value from the start point to the max value (b) is defined as the trough value (d), and the value from the start point to the min value (c) is defined as the time (e) taken from the start point to the trough value (d).

<Table 7>

|  | | Parameter | Description |
|---|---|---|---|
| (a) | | Time stand-to-sit | Time taken |
| (b) | | Peak_ stand-to-sit | Peak value |
| (c) | | Time peak_ stand-to-sit | Time taken from start point to peak value |
| (d) | | Trough_stand-to-sit | Trough value |
| (e) | | Time trough_stand-to-sit | Time taken from start point to trough value |
| (f) | | ax low frequency power | Low frequency power (0.5 to 3 Hz) |
| (g) | | ax high frequency power | High frequency power (3 to 8 Hz) |
| (h) | | ay low frequency power | Low frequency power (0.5 to 3 Hz) |
| (i) | | ay high frequency power | High frequency power (3 to 8 Hz) |
| (j) | | az low frequency power | Low frequency power (0.5 to 3 Hz) |
| (k) | | az high frequency power | High frequency power (3 to 8 Hz) |
| (l) | | svm low frequency power | Low frequency power (0.5 to 3 Hz) |

(continued)

| | Parameter | Description |
|---|---|---|
| (m) | svm high frequency power | High frequency power (3 to 8 Hz) |

So as to determine the frailty of the patient, generally, the conventional physical performance assessment device uses the SPPB that measures the patient's gait speed, chair stand, and balance to determine his or her physical performance. However, professionally trained personnel (hereinafter, referred to as medical staff) is needed in executing comprehensive geriatric assessment for older patients, and further, a lot of time is disadvantageously required for the assessment. Besides, the patient's physical performance is measured with the naked eye of the medical staff, and accordingly, the degree of accuracy in the assessment may be diminished. Further, the assessment results may be varied according to the patient's mood or condition upon the test, and the frailty of the patient is assessed through indirect data, thereby making the degree of accuracy in the assessment results deteriorated.

Further, the conventional physical performance assessment device is configured to attach a sensor to the lower limb of the body to collect the ground reaction force or to use two different force plates to collect ground reaction forces separated from each other. Accordingly, since the ground reaction forces for the left and right legs are collected, they have to be added to perform the patient's gait analysis, thereby disadvantageously making the work efficiency lowered. Besides, if the sensor is attached to the lower limb, the degree of accuracy in the test may become lower than that in the case where the sensor is attached to the upper body of the patient, thereby disadvantageously making it difficult to perform data analysis. Moreover, in the case where the ground reaction forces are collected through the force plates, poor portability may occur.

To the contrary, the physical performance assessment device according to the present invention is configured to allow only the motion acceleration sensor to be attached to the head, so that the patient's motions, which have been measured with the naked eye in the conventional practice, can be accurately analyzed. As shown in FIGs. 1a and 1b, the physical performance assessment device 100 attached to the patient's head can obtain the gait time points of the right and left legs and detect the gait parameters, so that the motions can be divided to thus perform the physical performance assessment with a minimum error rate.

According to the present invention, further, the patient's motion acceleration signals are collected and the gait motions are analyzed only through the motion acceleration sensor attached to the head (that is, only through the physical performance assessment device 100), thereby improving the assessment speed.

Moreover, the gait time points for the left and right legs can be distinguished from each other based on the motion acceleration collected from the motion acceleration sensor attached to the head, thereby increasing the conveniences of the assessment.

In addition, the physical performance assessment device 100 is configured to collect the up and down acceleration, the front and back acceleration, and/or the left and right acceleration so that the patient's motions can be completely analyzed to accurately detect his or her gait abnormality due to a disease. For example, the gait abnormality motion patterns of the patient having Parkinson's disease or alzheimer's disease can be detected, thereby previously preventing the accidents of the patient from happening. For example, the patients can be protected from fall accidents through the analysis of their gait abnormality motion patterns.

While the present invention has been described with reference to the particular illustrative embodiments, it is not to be restricted by the embodiments but only by the appended claims. It is to be appreciated that those skilled in the art can change or modify the embodiments without departing from the scope of the present invention. This application is intended to cover any variations, uses, or adaptations of the invention following the general principles thereof and including such departures from the present disclosure as come within known or customary practice in the art. It is intended that the specification and examples be considered as exemplary only, with a true scope of the invention being indicated by the following claims.

**Claims**

1. A physical performance assessment method using a physical performance assessment device (100) having a motion measurement module (110) for measuring a patient's motion acceleration signals through a motion acceleration sensor (111) attached to the patient's head and a motion analysis module (120) for analyzing the patient's physical performance based on the measured motion acceleration signals, the method comprising the steps of:

Collecting (101) the motion acceleration signals measured from the motion acceleration sensor (111) attached to the patient's head through the motion measurement module (110) and used to analyze the patient's motions; and

Deriving (S104) a plurality of motion parameters based on the collected motion acceleration signals through the motion analysis module (110),
wherein the step of deriving the plurality of motion parameters comprises the steps of:

executing (S102) a protocol for balance among the plurality of motion parameters comprising the steps of: collecting the patient's three-axis motion acceleration signals for side-by-side stance, tandem stance, and semi-tandem stance and detecting balance parameters capable of assessing the patient's balance performance from total acceleration signals where the three-axis motion acceleration signals are added;
executing (S102) a protocol for gait speed among the plurality of motion parameters comprising the steps of: detecting single stance and double stance from the motion acceleration signals to detect gait parameters from the detected results, wherein the gait parameters further comprise low frequency power in low frequency band and high frequency power in high frequency band, an a rate of the low frequency power with respect to the high frequency power is computed; and
executing (S102) a protocol for chair stand, including a stand-to-sit motion and a sit-to-stand motion among the plurality of motion parameters comprising the steps of: detecting a minimum peak value and a maximum peak value from the up and down acceleration and the total motion acceleration signals to detect stand parameters through the relation between the minimum peak value and the maximum peak value.

2. The physical performance assessment method according to claim 1, further comprising the step of determining, when the protocol for the balance is executed, whether a balance test is completed based on the magnitude of the total acceleration signals with respect to a preset threshold value.

3. The physical performance assessment method according to claim 2, wherein when the magnitude of the total acceleration signals is greater than the preset threshold value, it is determined that the test fails or the patient quits in the middle of the test, the time taken is recorded up to the time point where the magnitude of the total acceleration signals is greater than the threshold value, and scores are applied to the recorded time according to preset score ranges.

4. The physical performance assessment method according to claim 3, wherein the magnitude of the total acceleration signals is a difference between a maximum peak value and a minimum peak value in the total acceleration signals.

5. The physical performance assessment method according to claim 1, further comprising the steps of:

detecting the patient's gait time points based on the gait parameters;
sensing a gait start point and a gait finish point based on the gait time points; and
assessing the patient's gait speed based on the time taken from the gait start point and the gait finish point.

6. The physical performance assessment method according to claim 1, wherein while the protocol for the chair stand is executed, when the minimum peak value is sensed earlier than the maximum peak value in the up and down acceleration and the total motion acceleration signals so that a difference between the minimum peak value and the maximum peak value is greater than a preset threshold value, it is determined that a stand-to-sit motion is performed.

7. The physical performance assessment method according to claim 1, wherein while the protocol for the chair stand is executed, when the maximum peak value is sensed earlier than the minimum peak value in the up and down acceleration and the total motion acceleration signals so that a difference between the minimum peak value and the maximum peak value is greater than the preset threshold value, it is determined that a sit-to-stand motion is performed.

8. The physical performance assessment method according to any one of claims 6 and 7, wherein the stand parameters comprise stand-to-sit parameters for the stand-to-sit motion and sit-to-stand parameters for the sit-to-stand motion.

9. The physical performance assessment method according to claim 1, wherein the gait parameters comprise vertical ground reaction forces and peak and trough values of the vertical ground reaction forces.

10. The physical performance assessment method according to claim 1, wherein the rate of the low frequency power with respect to the high frequency power is calculated as a constant value by the following Mathematical expression 1,

<Mathematical expression 1>

$$\frac{\text{High-band frequency (square of total power in the 3-8Hz band)}}{\text{Low-band frequency (square of total power in the 0.5-3Hz band)}} = \text{Constant}$$

and the constant value is compared to a preset constant value.

11. A physical performance assessment device (100) comprising:

a motion measurement module (110) configured to collect a patient's motion acceleration signals for the patient's motion analysis through a motion acceleration sensor (111) attached to the patient's head; and
a motion analysis module (120) configured to derive a plurality of motion parameters based on the collected motion acceleration signals,
wherein the motion analysis module (110) is configured to execute a protocol for balance, a protocol for gait speed, and a protocol for chair stand, and is configured to collect, when the protocol for balance among the plurality of motion parameters is executed, the patient's three-axis motion acceleration signals for side-by-side stance, tandem stance, and semi-tandem stance to detect balance parameters capable of assessing the patient's balance performance from total acceleration signals where the three-axis motion acceleration signals are added, to detect, the protocol for gait speed among the plurality of motion parameters is executed, single stance and double stance from the motion acceleration signals to detect gait parameters from the detected results, wherein the gait parameters further comprise low frequency power in low frequency band and high frequency power in high frequency band, and according to a rate of the low frequency power with respect to the high frequency power, the patient's gait motion is evaluated, and to detect, when the protocol for chair stand, including a stand-to-sit motion and a sit-to-stand motion among the plurality of motion parameters is executed, a minimum peak value and a maximum peak value from the up and down acceleration and the total motion acceleration signals to detect stand parameters through the relation between the minimum peak value and the maximum peak value.

12. The physical performance assessment device according to claim 11, wherein when the magnitude of the total acceleration signals is greater than the preset threshold value, it is determined that the test fails or the patient quits in the middle of the test, the time taken is recorded up to the time point where the magnitude of the total acceleration signals is greater than the threshold value, and scores are applied to the recorded time according to preset score ranges.

13. The physical performance assessment device according to claim 11, wherein the magnitude of the total acceleration signals is a difference between a maximum peak value and a minimum peak value in the total acceleration signals.

14. The physical performance assessment device according to claim 11, wherein when the minimum peak value is sensed earlier than the maximum peak value in the up and down acceleration and the total motion acceleration signals so that a difference between the minimum peak value and the maximum peak value is greater than the preset threshold value, it is determined that a stand-to-sit motion is performed.

15. The physical performance assessment device according to claim 11, wherein when the maximum peak value is sensed earlier than the minimum peak value in the up and down acceleration and the total motion acceleration signals so that a difference between the minimum peak value and the maximum peak value is greater than the preset threshold value, it is determined that a sit-to-stand is performed.

16. The physical performance assessment device according to claim 11, further comprising a notification module for providing test explanations to the form recognized by the patient such as sound, video, or the like so that through the test explanations, the patient executes the protocol.

**Patentansprüche**

1. Verfahren zur Beurteilung der körperlichen Leistungsfähigkeit unter Verwendung einer Vorrichtung zur Beurteilung der körperlichen Leistungsfähigkeit (100) mit einem Bewegungsmessmodul (110) zum Messen der Bewegungsbeschleunigungssignale eines Patienten über einen am Kopf des Patienten angebrachten Bewegungsbeschleunigungssensor (111) und einem Bewegungsanalysemodul (120) zum Analysieren der körperlichen Leistungsfähigkeit des Patienten auf der Grundlage der gemessenen Bewegungsbeschleunigungssignale, wobei das Verfahren die folgenden Schritte umfasst:

Sammeln (101) der Bewegungsbeschleunigungssignale, die vom am Kopf des Patients angebrachten Bewegungsbeschleunigungssensor (111) durch das Bewegungsmessmodul (110) gemessen und zur Analyse der Bewegungen des Patienten verwendet werden; und
Ableiten (S104) mehrerer Bewegungsparameter auf der Grundlage der gesammelten Bewegungsbeschleunigungssignale durch das Bewegungsanalysemodul (110),
wobei der Schritt des Ableitens der mehreren Bewegungsparameter die folgenden Schritte umfasst:

Ausführen (S102) eines Protokolls für das Gleichgewicht unter den mehreren Bewegungsparametern, umfassend die folgenden Schritte: Sammeln der dreiachsigen Bewegungsbeschleunigungssignale des Patienten für den Seit-an-Seite-Stand, den Tandem-Stand und den Semi-Tandem-Stand und Erfassen von Gleichgewichtsparametern, mit denen die Gleichgewichtsleistungsfähigkeit des Patienten anhand der Gesamtbeschleunigungssignale beurteilt werden kann, bei denen die dreiachsigen Bewegungsbeschleunigungssignale addiert werden;
Ausführen (S102) eines Protokolls für die Ganggeschwindigkeit unter den mehreren Bewegungsparametern, umfassend die folgenden Schritte: Erfassen von Einzelstand und Doppelstand aus den Bewegungsbeschleunigungssignalen, um Gangparameter aus den erfassten Ergebnissen zu erfassen, wobei die Gangparameter ferner eine Niederfrequenzleistung im Niederfrequenzband und eine Hochfrequenzleistung im Hochfrequenzband umfassen und ein Verhältnis der Niederfrequenzleistung zur Hochfrequenzleistung berechnet wird; und
Ausführen (S102) eines Protokolls für das Aufstehen vom Stuhl, einschließlich einer Stand-zu-Sitz-Bewegung und einer Sitz-zu-Stand-Bewegung, unter den mehreren Bewegungsparametern, umfassend die folgenden Schritte: Erfassen eines minimalen Spitzenwerts und eines maximalen Spitzenwerts aus den Aufwärts- und Abwärtsbeschleunigungs- und Gesamtbewegungsbeschleunigungssignalen, um Standparameter durch die Beziehung zwischen dem minimalen Spitzenwert und dem maximalen Spitzenwert zu erfassen.

2. Verfahren zur Beurteilung der körperlichen Leistungsfähigkeit nach Anspruch 1, umfassend ferner den Schritt, bei Ausführung des Protokolls für das Gleichgewicht auf der Grundlage der Größe der Gesamtbeschleunigungssignale in Bezug auf einen voreingestellten Schwellenwert festzustellen, ob ein Gleichgewichtstest abgeschlossen ist.

3. Verfahren zur Beurteilung der körperlichen Leistungsfähigkeit nach Anspruch 2, wobei, wenn die Größe der Gesamtbeschleunigungssignale größer als der voreingestellte Schwellenwert ist, festgestellt wird, dass der Test fehlgeschlagen ist oder der Patient den Test vorzeitig abgebrochen hat, die benötigte Zeit bis zu dem Zeitpunkt aufgezeichnet wird, an dem die Größe der Gesamtbeschleunigungssignale größer als der Schwellenwert ist, und Punktzahlen gemäß voreingestellten Punktzahlbereichen auf die aufgezeichnete Zeit angewendet werden.

4. Verfahren zur Beurteilung der körperlichen Leistungsfähigkeit nach Anspruch 3, wobei die Größe der Gesamtbeschleunigungssignale eine Differenz zwischen einem maximalen Spitzenwert und einem minimalen Spitzenwert in den Gesamtbeschleunigungssignalen ist.

5. Verfahren zur Beurteilung der körperlichen Leistungsfähigkeit nach Anspruch 1, umfassend ferner die folgenden Schritte:

Erfassen der Gangzeitpunkte des Patienten auf der Grundlage der Gangparameter;
Erfassen eines Gangstartpunkts und eines Gangendpunkts auf der Grundlage der Gangzeitpunkte; und
Beurteilen der Ganggeschwindigkeit des Patienten auf der Grundlage der Zeit, die vom Gangstartpunkt bis zum Gangendpunkt benötigt wird.

6. Verfahren zur Beurteilung der körperlichen Leistungsfähigkeit nach Anspruch 1, wobei während der Ausführung des Protokolls für das Aufstehen vom Stuhl, wenn der minimale Spitzenwert früher als der maximale Spitzenwert in den Aufwärts- und Abwärtsbeschleunigungs- und Gesamtbewegungsbeschleunigungssignalen erfasst wird, so dass eine Differenz zwischen dem minimalen Spitzenwert und dem maximalen Spitzenwert größer als ein voreingestellter Schwellenwert ist, festgestellt wird, dass eine Stand-zu-Sitz-Bewegung durchgeführt wird.

7. Verfahren zur Beurteilung der körperlichen Leistungsfähigkeit nach Anspruch 1, wobei während der Ausführung des Protokolls für das Aufstehen vom Stuhl, wenn der maximale Spitzenwert früher als der minimale Spitzenwert in den Aufwärts- und Abwärtsbeschleunigungs- und Gesamtbewegungsbeschleunigungssignalen erfasst wird, so dass eine Differenz zwischen dem minimalen Spitzenwert und dem maximalen Spitzenwert größer als der voreingestellte Schwellenwert ist, festgestellt wird, dass eine Sitz-zu-Stand-Bewegung durchgeführt wird.

8. Verfahren zur Beurteilung der körperlichen Leistungsfähigkeit nach einem der Ansprüche 6 und 7, wobei die Standparameter Stand-zu-Sitz-Parameter für die Stand-zu-Sitz-Bewegung und Sitz-zu-Stand-Parameter für die Sitz-zu-Stand-Bewegung umfassen.

9. Verfahren zur Beurteilung der körperlichen Leistungsfähigkeit nach Anspruch 1, wobei die Gangparameter vertikale Bodenreaktionskräfte und Spitzen- und Talwerte der vertikalen Bodenreaktionskräfte umfassen.

10. Verfahren zur Beurteilung der körperlichen Leistungsfähigkeit nach Anspruch 1, wobei das Verhältnis der Niederfrequenzleistung zur Hochfrequenzleistung als konstanter Wert durch den folgenden mathematischen Ausdruck 1 berechnet wird,

$$< \text{Mathematischer Ausdruck 1} >$$

$$\frac{\text{Hochbandfrequenz (Quadrat der Gesamtleistung im Band von 3 bis 8 Hz)}}{\text{Niederfrequenzband (Quadrat der Gesamtleistung im Band von 0,5 bis 3 Hz)}} = \text{Konstant}$$

wobei der konstante Wert mit einem voreingestellten konstanten Wert verglichen wird.

11. Vorrichtung zur Beurteilung der körperlichen Leistungsfähigkeit (100), umfassend:

ein Bewegungsmessmodul (110), das dazu konfiguriert ist, die Bewegungsbeschleunigungssignale eines Patienten für die Bewegungsanalyse des Patienten über einen am Kopf des Patienten angebrachten Bewegungsbeschleunigungssensor (111) zu sammeln; und
ein Bewegungsanalysemodul (120), das dazu konfiguriert ist, auf der Grundlage der gesammelten Bewegungsbeschleunigungssignale mehrere Bewegungsparameter abzuleiten,
wobei das Bewegungsanalysemodul (110) dazu konfiguriert ist, ein Protokoll für das Gleichgewicht, ein Protokoll für die Ganggeschwindigkeit und ein Protokoll für das Aufstehen vom Stuhl auszuführen, und so konfiguriert ist, dass es bei Ausführung des Protokolls für das Gleichgewicht unter den mehreren Bewegungsparametern dreiachsige Bewegungsbeschleunigungssignale des Patienten für den Seit-an-Seite-Stand, den Tandem-Stand und den Semi-Tandem-Stand sammelt, um Gleichgewichtsparameter zu erfassen, mit denen die Gleichgewichtsleistungsfähigkeit des Patienten anhand der Gesamtbeschleunigungssignale beurteilt werden kann, bei denen die dreiachsigen Bewegungsbeschleunigungssignale addiert werden, bei Ausführung des Protokolls für die Ganggeschwindigkeit unter den mehreren Bewegungsparametern Einzelstand und Doppelstand aus den Bewegungsbeschleunigungssignalen erfasst, um aus den erfassten Ergebnissen Gangparameter zu erfassen, wobei die Gangparameter ferner eine Niederfrequenzleistung im Niederfrequenzband und eine Hochfrequenzleistung im Hochfrequenzband umfassen, wobei entsprechend einem Verhältnis der Niederfrequenzleistung zur Hochfrequenzleistung die Gangbewegung des Patienten bewertet wird, und bei Ausführung des Protokolls für das Aufstehen vom Stuhl, einschließlich einer Stand-zu-Sitz-Bewegung und einer Sitz-zu-Stand-Bewegung, unter den mehreren Bewegungsparametern, einen minimalen Spitzenwert und einen maximalen Spitzenwert aus den Aufwärts- und Abwärtsbeschleunigungs- und den Gesamtbewegungsbeschleunigungssignalen erfasst, um Standparameter durch die Beziehung zwischen dem minimalen Spitzenwert und dem maximalen Spitzenwert zu erfassen.

12. Vorrichtung zur Beurteilung der körperlichen Leistungsfähigkeit nach Anspruch 11, wobei, wenn die Größe der Gesamtbeschleunigungssignale größer als der voreingestellte Schwellenwert ist, festgestellt wird, dass der Test fehlgeschlagen ist oder der Patient den Test vorzeitig abgebrochen hat, die benötigte Zeit bis zu dem Zeitpunkt aufgezeichnet wird, an dem die Größe der Gesamtbeschleunigungssignale größer als der Schwellenwert ist, und Punktzahlen gemäß voreingestellten Punktzahlbereichen auf die aufgezeichnete Zeit angewendet werden.

**13.** Vorrichtung zur Beurteilung der körperlichen Leistungsfähigkeit nach Anspruch 11, wobei die Größe der Gesamtbeschleunigungssignale eine Differenz zwischen einem maximalen Spitzenwert und einem minimalen Spitzenwert in den Gesamtbeschleunigungssignalen ist.

**14.** Vorrichtung zur Beurteilung der körperlichen Leistungsfähigkeit nach Anspruch 11, wobei, wenn der minimale Spitzenwert früher als der maximale Spitzenwert in den Aufwärts- und Abwärtsbeschleunigungs- und Gesamtbewegungsbeschleunigungssignalen erfasst wird, so dass eine Differenz zwischen dem minimalen Spitzenwert und dem maximalen Spitzenwert größer als der voreingestellte Schwellenwert ist, festgestellt wird, dass eine Stand-zu-Sitz-Bewegung durchgeführt wird.

**15.** Vorrichtung zur Beurteilung der körperlichen Leistungsfähigkeit nach Anspruch 11, wobei, wenn der maximale Spitzenwert früher als der minimale Spitzenwert in den Aufwärts- und Abwärtsbeschleunigungs- und Gesamtbewegungsbeschleunigungssignalen erfasst wird, so dass eine Differenz zwischen dem minimalen Spitzenwert und dem maximalen Spitzenwert größer als der voreingestellte Schwellenwert ist, festgestellt wird, dass eine Sitz-zu-Stand-Bewegung durchgeführt wird.

**16.** Vorrichtung zur Beurteilung der körperlichen Leistungsfähigkeit nach Anspruch 11, umfassend ferner ein Benachrichtigungsmodul, um Testbeschreibungen in einer vom Patienten erkannten Form, wie beispielsweise Ton, Video oder dergleichen, bereitzustellen, so dass der Patient anhand der Testbeschreibungen das Protokoll ausführt.

**Revendications**

**1.** Procédé d'évaluation de performances physiques à l'aide d'un dispositif d'évaluation de performances physiques (100) comportant un module de mesure de mouvement (110) pour mesurer des signaux d'accélération de mouvement d'un patient au monyen d'un capteur d'accélération de mouvement (111) fixé à la tête du patient, et d'un module d'analyse de mouvement (120) pour analyser les performances physiques du patient en fonction des signaux d'accélération de mouvement mesurés, le procédé comprenant les étapes suivantes :

collecter (101) les signaux d'accélération de mouvement d'un patient mesurés par le capteur d'accélération de mouvement (111) fixé à la tête du patient au monyen du module de mesure de mouvement (110) et utilisés pour analyser les mouvements du patient ; et
extraire (S104) une pluralité de paramètres de mouvements à partir des signaux d'accélération de mouvement collectés au monyen du module d'analyse de mouvement (110),
dans lequel l'étape d'extraction de la pluralité de paramètres de mouvement comprend les étapes suivantes :

exécuter (S102) un protocole d'équilibre parmi la pluralité de paramètres de mouvement comprenant les étapes consistant à : collecter les signaux d'accélération de mouvement à trois axes du patient pour une position côte à côte, une position en tandem et une position en semi-tandem et détecter des paramètres d'équilibre permettant d'évaluer les performances d'équilibre du patient à partir de signaux d'accélération totale où les signaux d'accélération de mouvement à trois axes sont additionnés ;
exécuter (S102) un protocole de vitesse de marche parmi la pluralité de paramètres de mouvement comprenant les étapes consistant à : détecter une position d'appui simple et une position d'appui double à partir des signaux d'accélération de mouvement pour détecter des paramètres de marche à partir des résultats détectés, dans lequel les paramètres de marche comprennent en outre une puissance à basse fréquence dans une bande à basse fréquence et une puissance à haute fréquence dans une bande à haute fréquence, et un taux de puissance à basse fréquence par rapport à la puissance à haute fréquence est calculé ; et
exécuter (S102) un protocole pour se lever d'une chaise, comprenant un mouvement de position debout à position assise et un mouvement de position assise à position debout parmi la pluralité de paramètres de mouvement comprenant les étapes consistant à : détecter une valeur de pic minimale et une valeur de pic maximale à partir des signaux d'accélération de haut en bas et d'accélération totale de mouvement pour détecter les paramètres de position debout par l'intermédiaire de la relation entre la valeur de pic minimale et la valeur de pic maximale.

**2.** Procédé d'évaluation de performances physiques selon la revendication 1, comprenant en outre l'étape consistant à déterminer, lorsque le protocole de l'équilibre est exécuté, si un test d'équilibre est terminé sur la base de l'amplitude des signaux d'accélération totale par rapport à une valeur de seuil prédéfinie.

3. Procédé d'évaluation de performances physiques selon la revendication 2, dans lequel lorsque l'amplitude des signaux d'accélération totale est supérieure à la valeur de seuil prédéfinie, il est déterminé que le test échoue ou que le patient quitte au milieu du test, le temps pris est enregistré jusqu'au moment où l'amplitude des signaux d'accélération totale est supérieure à la valeur de seuil, et des scores sont appliqués au temps enregistré en fonction des plages de scores prédéfinies.

4. Procédé d'évaluation de performances physiques selon la revendication 3, dans lequel l'amplitude des signaux d'accélération totale est une différence entre une valeur de pic maximale et une valeur de pic minimale dans les signaux d'accélération totale.

5. Procédé d'évaluation de performances physiques selon la revendication 1, comprenant en outre les étapes suivantes :

   détecter les moments de marche du patient en fonction des paramètres de marche ;
   détecter un point de début de marche et un point de fin de marche en fonction des moments de marche ; et
   évaluer la vitesse de marche du patient en fonction du temps écoulé entre le point de début de marche et le point de fin de marche.

6. Procédé d'évaluation de performances physiques selon la revendication 1, dans lequel, tandis que le protocole pour se lever d'une chaise est exécuté, lorsque la valeur de pic minimale est détectée plus tôt que la valeur de pic maximale dans les signaux d'accélération de haut en bas et d'accélération totale de mouvement de sorte qu'une différence entre la valeur de pic minimale et la valeur de pic maximale est supérieure à une valeur de seuil prédéfinie, il est déterminé qu'un mouvement de position debout à position assise est effectué.

7. Procédé d'évaluation de performances physiques selon la revendication 1, dans lequel, tandis que le protocole pour se lever d'une chaise est exécuté, lorsque la valeur de pic maximale est détecté plus tôt que la valeur de pic minimale dans les signaux d'accélération de haut en bas et d'accélération totale de mouvement de sorte qu'une différence entre la valeur de pic minimale et la valeur de pic maximale est supérieure à la valeur de seuil prédéfinie, il est déterminé qu'un mouvement de position assise à position debout est effectué.

8. Procédé d'évaluation de performances physiques selon l'une quelconque des revendications 6 et 7, dans lequel les paramètres de position deboutcomprennent des paramètres de position debout à position assise pour le mouvement de position debout à position assise et des paramètres de position assise à position debout pour le mouvement de position assise à position debout.

9. Procédé d'évaluation de performances physiques selon la revendication 1, dans lequel les paramètres de marche comprennent des forces de réaction verticales au sol, ainsi que des valeurs de pic et de creux des forces de réaction verticales au sol.

10. Procédé d'évaluation de performances physiques selon la revendication 1, dans lequel le taux de puissance à basse fréquence par rapport à la puissance à haute fréquence est calculé comme valeur constante par l'expression mathématique 1 suivante,

<Expression mathématique 1>

$$\frac{\text{Fréquence de haute bande (carrée de la puissance totale dans la bande de 3 - 8 Hz)}}{\text{Fréquence de basse bande (carrée de la puissance totale dans la bande de 0.5 - 3 Hz)}} = \text{Constante}$$

, et la valeur constante est comparée à une valeur constante prédéfinie.

11. Dispositif d'évaluation de performances physiques (100) comprenant :

   un module de mesure de mouvement (110) configuré pour collecter les signaux d'accélération de mouvement d'un patient pour l'analyse de mouvement du patient au moyen d'un capteur d'accélération de mouvement (111) fixé à la tête du patient ; et

un module d'analyse de mouvement (120) configuré pour extraire une pluralité de paramètres de mouvement à partir des signaux d'accélération de mouvement collectés,

dans lequel le module d'analyse de mouvement (110) est configuré pour exécuter un protocole d'équilibre, un protocole de vitesse de marche et un protocole pour se lever d'une chaise, et est configuré pour collecter, lorsque le protocole d'équilibre parmi la pluralité de paramètres de mouvement est exécuté, les signaux d'accélération de mouvement à trois axes du patient pour une position côte à côte, une position en tandem et une position en semi-tandem afin de détecter des paramètres d'équilibre permettant d'évaluer les performances d'équilibre du patient à partir des signaux d'accélération totale où les signaux d'accélération de mouvement à trois axes sont additionnés, pour détecter, lorsque le protocole de vitesse de marche parmi la pluralité de paramètres de mouvement est exécuté, une position d'appui simple et une position d'appui double à partir des signaux d'accélération de mouvement pour détecter des paramètres de marche à partir des résultats détectés, dans lequel les paramètres de marche comprennent en outre une puissance à basse fréquence dans une bande à basse fréquence et une puissance à haute fréquence dans une bande à haute fréquence, et en fonction d'un taux de puissance basse fréquence par rapport à la puissance haute fréquence, le mouvement de marche du patient est évalué, et

pour détecter, lorsque le protocole pour se lever d'une chaise, comprenant un mouvement de position debout à position assise et un mouvement de position assise à position debout parmi la pluralité de paramètres de mouvement sont exécutés, une valeur de pic minimale et une valeur de pic maximale à partir des signaux d'accélération de haut en bas et d'accélération totale de mouvement pour détecter les paramètres de position debout par l'intermédiaire de la relation entre la valeur de pic minimale et la valeur de pic maximale.

12. Dispositif d'évaluation de performances physiques selon la revendication 11, dans lequel lorsque l'amplitude des signaux d'accélération totale est supérieure à la valeur de seuil prédéfinie, il est déterminé que le test échoue ou que le patient quitte au milieu du test, le temps pris est enregistré jusqu'au moment où l'amplitude des signaux d'accélération totale est supérieure à la valeur de seuil, et des scores sont appliqués au temps enregistré en fonction des plages de scores prédéfinies.

13. Dispositif d'évaluation de performances physiques selon la revendication 11, dans lequel l'amplitude des signaux d'accélération totale est une différence entre une valeur de pic maximale et une valeur de pic minimale dans les signaux d'accélération totale.

14. Procédé d'évaluation de performances physiques selon la revendication 11, dans lequel, tandis que la valeur de pic minimale est détectée plus tôt que la valeur de pic maximale dans les signaux d'accélération de haut en bas et d'accélération totale de mouvement de sorte qu'une différence entre la valeur de pic minimale et la valeur de pic maximale est supérieure à une valeur de seuil prédéfinie, il est déterminé qu'un mouvement de position debout à position assise est effectué.

15. Procédé d'évaluation de performances physiques selon la revendication 11, dans lequel, tandis que la valeur de pic maximale est détectée plus tôt que la valeur de pic minimale dans les signaux d'accélération de haut en bas et d'accélération totale de mouvement de sorte qu'une différence entre la valeur de pic minimale et la valeur de pic maximale est supérieure à une valeur de seuil prédéfinie, il est déterminé qu'un mouvement de position assise à position debout est effectué.

16. Dispositif d'évaluation de performances physiques selon la revendication 11, comprenant en outre un module de notification permettant de fournir des explications de test sous la forme reconnue par le patient, telle qu'un son, une vidéo ou des similaires, de sorte que, à travers les explications de test, le patient exécute le protocole.

**FIG. 1a**

**FIG. 1b**

FIG. 2

100

130

113 — Communication module
Bluetooth  NFC/RFID
ZIGBEE  Wi-Fi

112 — Sensor control module

111 — Sensor module
Acceleration sensor
Gyro sensor

110

125 — Stand parameter detection module

126 — Communication module
Bluetooth  NFC/RFID
ZIGBEE  Wi-Fi

127 — User interface module

128 — Analysis control module

121 — Acceleration collection module

122 — Balance parameter detection module

123 — Gait time point detection module

124 — Gait parameter detection module

120

140

```
                ┌──────────────────────────────────┐
                │                                  │
                │      Data collection step        │ ～ S101
                │                                  │
                └──────────────────────────────────┘
                                │
                                ▼
                ┌──────────────────────────────────┐
                │                                  │
                │   SPPB protocol execution step   │ ～ S102
                │                                  │
                └──────────────────────────────────┘
                                │
                                ▼
                ┌──────────────────────────────────┐
                │                                  │
                │  Protocol motion recognition step│ ～ S103
                │                                  │
                └──────────────────────────────────┘
                                │
                                ▼
                ┌──────────────────────────────────┐
                │                                  │
                │  Motion parameter deriving step  │ ～ S104
                │                                  │
                └──────────────────────────────────┘
                                │
                                ▼
                ┌──────────────────────────────────┐
                │                                  │
                │  SPPB test result acquiring step │ ～ S105
                │                                  │
                └──────────────────────────────────┘
                      │                   │
          ┌───────────┘                   └───────────┐
          ▼                                           ▼
  ┌──────────────────┐                   ┌──────────────────────┐
  │ Storing SPPB test│                   │  Transmission of test│
  │   result in DB   │──────────────────▶│ result to medical staff│
  └──────────────────┘                   └──────────────────────┘
```

**FIG. 3**

FIG. 4

FIG. 5a

**FIG. 5b**

**FIG. 5b**

EP 4 098 187 B1

**FIG. 6**

FIG. 7

FIG. 8

EP 4 098 187 B1

| Gait acceleration collection step | S410 |

| Gait time point detection step | S420 |

| Gait parameter detection step | S430 |

**FIG. 9**

32

**FIG. 10**

**Pre-test**
Participants fold their arms across their chest
and try to stand up once from a chair

unable ----→ Stop (0 pt)

able

**5 repeats**
Measures the time required to perform five rises
from a chair to an upright position as fast as
possible without the use of the arms

≤11.19 sec          4 pt
11.20-13.69 sec     3 pt
13.70-16.69 sec     2 pt
>16.7 sec           1 pt
>60 sec or unable   0 pt

**FIG. 11**

EP 4 098 187 B1

FIG. 12

**FIG. 13**

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20190098635 A
- KR 20190015851 A
- KR 20190115978 A
- US 2017258370 A1
- KR 102055661